# FASCICULE DE BREVET EUROPEEN

(11) **EP 0 489 138 B1**
(45) Date de publication et mention de la délivrance du brevet: **13.08.1997**
(21) Numéro de dépôt: 91911850.5
(22) Date de dépôt: 20.06.1991
(51) Int. Cl.: A61K 39/118

(54) **Composition vaccinale destinée au traitement d'affections humaines à Chlamydia**
Impfstoff zur Behandlung menschlicher Chlamydienkrankheiten
Vaccine composition for treating human Chlamydia infections

(30) Priorité: 22.06.1990 CH 2083/90; 26.10.1990 CH 3431/90
(43) Date de publication de la demande: 10.06.1992
(73) Titulaire: GOUCHET, Frank, F-45450 Donnery (FR); LIOTET, Serge, F-75011 Paris (FR); CATALAN, François, F-75018 Paris (FR)
(72) Inventeur: LIOTET, Serge, F-75011 Paris (FR); CATALAN, François, F-75018 Paris (FR)
(74) Mandataire: Leger, Jean-François
(86) Numéro de dépôt international: EP9101168
(87) Numéro de publication internationale: WO9200096

(56) Documents cités:
- FR-A- 2 507 479
- GB-A- 2 092 443
- Vaccine, vol. 5, no. 1, mars 1987, Butterworth & Co. (Publishers) Ltd, (Guildford, Surrey, GB), M. Plommet et al.: "Simultaneous vaccination by three living attenuated strains of Brucella, Salmonella and Chlamydia in mice", pages 27-32, voir l'article en entier
- Embase, abrégé no. 82041115, A.P. Johnson et al.: "Immunity to reinfection of the genital tract of marmosets with Chlamydia trachomatis", & Br. J. Exp. Pathol. (England), 1981, 62/6 (606-613), voir abrégé en entier
- Journal of Immunology, 128, 1982, 1083-9
- Infection and Immunity, 58(1), 1990, 93-7

## Description

L'invention a pour objet une composition destinée au traitement préventif ou curatif par stimulation antigénique orale d'affections oculaires, oculo- ou urogénitales humaines de type chlamydioses, par exemple le trachome.

Les muqueuses, qui présentent une vaste surface en contact avec le milieu extérieur, sont l'objet d'agressions constantes et possèdent un système de défense très puissant pour faire face à ces attaques, avec des facteurs antimicrobiens non spécifiques et spécifiques. Les systèmes spécifiques de protection des muqueuses conjonctivales font partie du large système MALT (mucosa associated lymphoïd tissue) comprenant pour l'essentiel le système GALT associé au tube digestif et des systèmes annexes, dont le système BALT associé aux bronches ou le système CALT (conjonctiva associated lymphoïd tissue).

L'immunité spécifique des muqueuses est due essentiellement aux immunoglobulines secrétoires (IgAs) qui sont synthétisées sur place par des plasmocytes provenant de lymphocytes B activés qui ont reçu une stimulation antigénique dans n'importe quelle partie des muqueuses, in situ ou à distance. Il a été démontré, par des méthodes chimiques et par immunofluorescence, qu'une partie des IgAs sécrétoires se fixe sur le mucus conjonctival et à la surface des cellules épithéliales, et forme ainsi une barrière continue d'anticorps qui s'oppose aux agressions extérieures.

Dans un article intitulé "Changes in conjunctival lymphocite populations induced by oral immunisation with Chlamydia trachomatis" - Current Eye Research, Vol.5, No. 12, 1986, 973-979 - il est fait mention d'une immunisation partielle au niveau de la conjonctive oculaire, sur le modèle animal retenu à fin d'expérimentation (singe). L'analyse des résultats montre cependant que ceux-ci sont loin d'être satisfaisants. En effet :
- une tentative d'immunisation par voie orale à l'aide de sérotype B vivant de Chlamydia trachomatis n'induit aucune immunisation au niveau de la conjonctive oculaire, comme le confirme un challenge avec le même sérotype;
- l'immunisation par voie orale, à l'aide de sérotype L₂ vivant (lymphogranulomatose) de Chlamydia trachomatis n'induit qu'une immunisation partielle de la conjonctive oculaire, tel que le confirme un challenge avec le sérotype B (trachome);
- une tentative d'immunisation par voie orale au moyen de sérotype L₂ tué n'induit aucune immunisation de la conjonctive oculaire, mais au contraire, affaiblit le système conjonctival oculaire.

On constate ainsi qu'une stimulation antigénique orale, à l'aide de tels sérotypes vivants n'est que faible, voire inexistante au niveau de la conjonctive oculaire. Rien n'indique en outre qu'au vu de leur virulence, les sérotypes vivants n'entraînent pas d'effets secondaires indésirables.

Pour ce qui est des germes tués, la voie proposée est sans issue puisqu'elle conduit au contraire à un affaiblissement du système immunitaire local, au niveau de la conjonctive oculaire pour le moins. En outre, concernant les affections de type uro-génital aucune déduction n'est possible de telles expérimentations.

Il apparaît donc, dans le cas des affections à Chlamydia, que la stimulation antigénique orale n'est pas la voie appropriée, ce que confirme d'ailleurs la littérature scientifique, qui fait essentiellement état de la recherche ou de l'amélioration de compositions vaccinales injectables par voie sous-cutanée ou intra-musculaire.

Il a été découvert, de façon surprenante, que grâce à la présente invention l'on pouvait avantageusement surmonter de tels obstacles en proposant, contrairement aux préjugés établis, une composition vaccinale particulièrement efficace dans le traitement d'affections à Chlamydiae.

L'invention découle de la découverte que des germes lyophilisés de l'espèce Chlamydia trachomatis sont, en fait, des germes tués qui ont perdu toute leur virulence, mais conservé leur pouvoir immunogène. Il en va ainsi tout particulièrement pour les sérotypes A à C, respectivement D à K de l'espèce susmentionnée qui, administrés convenablement par voie orale, induisent une immunisation satisfaisante de la conjonctive oculaire aussi bien que celle des muqueuses uro-génitales.

L'invention a pour objet une composition vaccinale destinée au traitement préventif ou curatif par stimulation antigénique orale d'affections oculaires, oculo- ou urogénitales humaines de type chlamydioses, telle que définie à la revendication 1.

Dans le cas de telles affections, il est maintenant démontré que la stimulation la plus appropriée pour la formation d'anticorps de type IgAs au niveau des épithélias conjonctivaux ou uro-génitaux se situe au niveau de l'intestin.

Dans le traitement préventif ou curatif d'affections oculaires humaines comme le trachome, on obtient de bons résultats à l'aide d'une telle composition préparée à l'aide d'au moins un des sérotypes A à C, ou selon les cas tous ces sérotypes, de l'espèce Chlamydia trachomatis.

Dans le cas d'affections oculo- ou uro-génitales humaines de type chlamydioses, on utilise de préférence une composition préparée à l'aide d'au moins un des sérotypes D à K ou, selon les cas, l'ensemble de ces sérotypes, de l'espèce Chlamydia trachomatis.

Selon l'invention, la composition peut avantageusement contenir un diluant ou excipient inerte, pharmaceutiquement acceptable et, le cas échéant, un adjuvant ou stimulant immunologique adéquat, ou encore un adjuvant stimulant l'activité des macrophages. Une telle énumération n'est pas exhaustive.

La composition selon l'invention s'administre par voie orale, elle peut se présenter sous une forme galénique gastro-résistante telle une gélule ou un comprimé gastro-résistant, sous forme de comprimé à faire fondre dans la bouche ou encore sous forme de microsphères, nanosphères, microcapsules ou nanocapsules biodégradables, ou encore sous forme de vésicules lipidiques ou de liposomes. Il est du ressort de l'homme du métier de déterminer la forme d'administration la plus adéquate.

Pour obtenir l'immunisation souhaitée, il convient d'administrer la composition de manière répétée, sur une période de 15 à 30 jours et d'effectuer un rappel après 3 mois, le cas échéant après 1 an. Par exemple, on peut administrer la dose prescrite à J₁, J₂, J₃ puis à J₁₅, J₁₆ et J₁₇ et effectuer ensuite un rappel à 90 jours puis au bout d'un an, ou encore à J₁, J₁₅ et J₃₀, les rappels subséquents étant également effectués à 90 jours, puis au bout d'un an. Bien entendu, tout autre rythme d'administration peut être envisagé.

L'efficacité de compositions conformes à l'invention peut être illustrée comme suit. Cette illustration n'est en aucun cas limitative.

### Expérimentation sur le lapin

**a.1** On prépare une suspension antigénique à partir de culture de Chlamydia trachomatis sérotype D en procédant comme suit : les antigènes sont obtenus en culture de Chlamydia sur cellules McCOY ou HELA 229, et comportent un mélange de corps réticulés et de corps élémentaires. Ces cellules sont remises en suspension et ultrasoniquées ou séparées par tout autre système mécanique.
Les anticorps sont recherchés et dosés à partir du 8ème jour dans le sang et les larmes, l'absence d'anticorps ayant été vérifiée avant toute injection. Les anticorps se positivent dès le 8ème jour à un titre faible dans le sérum (1/4 à 1/8) et se précisent franchement dès le 15ème jour (3ème détermination : J-₁, J₈, J₁₅).
Le tableau suivant donne les titres observés pour cinq lapins répartis dans les cages 114, 115, 116, 117 et 119, en fonction du temps écoulé depuis l'immunisation :
(voir tableau à la page 6)
On a ainsi pu observer que l'ingestion, trois jours consécutifs à deux reprises, de particules chlamydiennes vivantes entraîne chez le lapin l'apparition d'anticorps spécifiques à un titre élevé, aussi bien dans le sérum que dans les larmes.

| **LAPIN** | | **114** | **115** | **116** | **117** | **119** |
|---|---|---|---|---|---|---|
| avant immunisatio n larmes et sérum | | 0 | 0 | 0 | 0 | 0 |
| J₁₂ | LARMES | 1/4 | 1/8 | 1/8 | douteux | 1 |
| | SERUM | 1/16 | 1/256 | 1/128 | 1/16 | 1/4 |
| J₂₀ | LARMES | 1/128 | 1/512 | 1/128 | 1/32 | 1/8 |
| | SERUM | * | * | * | * | * |
| J₃₀ | LARMES | 1/1024 | 1/16384 | 1/16384 | 1/1024 | 1/128 |
| | SERUM | 1/2048 | 1/32768 | 1/16384 | 1/8192 | 1/1024 |
| J₁₂₀ | LARMES | 1/256 | 1/256 | 1/64 | * | * |
| | SERUM | 1/4096 | 1/8192 | 1/1024 | * | * |
| * : Titrage non effectué | | | | | | |

**a.2** Des expérimentations identiques ont été menées sur le lapin avec les sérotypes A et B ainsi que A, B et C pour ce qui concerne le trachome et avec des sérotypes D à K pour ce qui concerne les conjonctivites à inclusion.
Chlamydiae trachomatis ne présentant aucune pathogénécité par voie buccale et digestive, la forme active vivante donne d'excellents résultats.
Des résultats comparables ont été obtenus avec des bactéries atténuées par du formol à 1%, durant une heure à 4^{o}C.
- Absorption:: J₁, J₂, J₃, J₁₅, J₁₆, J₁₇ - immunisation vers J₂₅
- Absorption:: J₁, J₁₅, J₃₀ - immunisation vers J₃₅

**a.3** On a préparé séparément deux suspensions antigéniques à partir d'une culture de Chlamydia trachomatis sérotype D conformément au processus décrit sous a.1.

La première suspension, contenant les germes vivants, a été utilisée telle que pour l'expérimentation. La seconde suspension a été soumise à lyophilisation, ce qui a conduit à l'obtention de germes tués comme l'ont confirmé des essais de culture subséquents.

La mise en évidence de l'apparition d'anticorps a été effectuée en parallèle sur deux groupes de 5 lapins chacun, l'un recevant les germes vivants, l'autre les germes lyophilisés. L'administration des antigènes Chlamydia a été effectuée par tubage gastrique comme précédemment et les prélèvements de sérum ont été pratiqués à J₀, J₇, J₁₄, J₂₁ et J₂₈.

Selon la technique du Western-Blot, les échantillons de sérum prélevés comme ci-dessus, montrent l'apparition d'anticorps anti-Chlamydia à partir de J₁₄, ceux-ci atteignant un maximum de variété à J₂₁. L'expérimentation permet en outre de préciser que les résultats sont identiques, qu'ils soient obtenus à partir de germes vivants ou lyophilisé, tant du point de vue quantitatif que qualitatif.

### Expérimentation sur la singe

Six singes lynomolgus adultes femelles, après une période d'acclimatation de 4 semaines, ont été répartis en deux groupes distincts :
- Témoins: : no. 01, 06, 09
- Tests: : no. 03, 04, 05

La composition vaccinale consiste en une suspension dans de l'eau stérile de germes lyophlisés de Chlamydia trachomatis sérotype D. Le titre infectant est de 10⁸ germes/ml.

La composition vaccinale a été administrée par voie orale à chacun des sujets du groupe Tests, à raison de 1 ml de suspension par animal, selon le calendrier suivant : J₁ J₂, J₃, J₁₅, J₁₆, J₁₇, un rappel étant prévu à J₉₁, J₉₂, J₉₃. Pendant le même temps, chacun des sujets témoins reçoit un ml d'eau stérile.

L'efficacité de la vaccination se détermine par la mesure du titre d'anticorps sérique suite à des prélévements sanguins réguliers, ainsi que par la détermination de la présence d'IgAs dans les larmes et dans les sécrétions vaginales. Avant le début de l'expérimentation, on s'est en outre assuré, au moyen des mêmes prélévements, de la présence éventuelle d'anticorps spécifiques.

Le tableau ci-après rassemble les résultats observés. Les chiffres donnent le taux sérique des anticorps, la présence d'IgAs dans les larmes (o = oeil) et les sécrétions vaginales (v = vagin) étant confirmée par le signe "+" (mesure semi-quantitative).
(voir tableau à la page 10)

Bien que, comme on pouvait s'y attendre, la réponse individuelle varie d'un sujet à l'autre, les animaux tests présentent tous une réponse immunitaire significative à la stimulation antigénique orale, au niveau des anticorps circulants comme au niveau des IgAs.

Cette constatation est en outre renforcée par le fait que les sujets vaccinés résistent bien à un challenge oculaire ou vaginal effectué après vaccination, par inoculation d'une souche Chlamydia trachomatis virulente, sérotype D.

| **No** | **Jour 0** | **Jour 8** | **Jour 15** | **Jour 21** | **Jour 30** | **Jour 35** | **Jour 45** |
|---|---|---|---|---|---|---|---|
| 01 | o = -32 | / | / | / | o = -32 | o = -32 | o = -64 |
| | v = - | | | | v = - | v = - | v = ± |
| 06 | o = -16 | / | / | / | o = -<16 | o = -<16 | o = -<16 |
| | v = - | | | | v = - | v = - | v = - |
| 09 | o = -env. 16 | / | / | / | o = -<16 | o = -<16 | o = -64 |
| | v = - | | | | v = - | v = - | v = - |
| 03 | o = -env. 16 | o = -16 | o = -128 | o = -64 | o = + 64 | o = -32 | o = -16 |
| | v = - | v = - | v = ± | v = + | v = + | v = + | v = - |
| 04 | o = -env. 16 | o = -16 | o =-256 | o =++ 1024 | o =++ 1024 | o =++ 1024 | o=+++ 512 |
| | v = - | v = - | v = - | v = - | v =++ | v =++ | v = - |
| 05 | o = -32 | o = -env. 16 | o = ± 64 | o = -64 | o = -64 | o = -32 | o = -64 |
| | v = - | v = - | v = ± | v = - | v = ± | v = - | v = - |

## Revendications

1. Composition vaccinale destinée au traitement préventif ou curatif par stimulation antigénique orale d'affections oculaires, oculo- ou uro-génitales humaines de type chlamydioses, caractérisée en ce qu'elle contient en mélange des corps réticulés et des corps élémentaires lyophilisés de Chlamydia trachomatis présentant au moins l'un des sérotypes A à K de ladite espèce.

2. Composition selon la revendication 1 destinée au traitement d'affections oculaires humaines tel le trachome, caractérisée en ce qu'elle contient au moins un des sérotypes A à C de l'espèce Chlamydia trachomatis.

3. Composition selon la revendication 1 destinée au traitement d'affections oculo- ou uro-génitales humaines de type chlamydioses, caractérisée en ce qu'elle contient au moins un des sérotypes D à K de l'espèce Chlamydia trachomatis.

4. Composition selon l'une des revendications 1 à 3, caractérisée en ce qu'elle contient outre un excipient inerte, un adjuvant ou stimulant immunologique ou encore un adjuvant stimulant l'activité des macrophages.

5. Composition selon l'une des revendications 1 à 4, caractérisée en ce qu'elle se présente sous une forme galénique orale gastro-résistante.

6. Composition selon l'une des revendications 1 à 5, caractérisée en ce qu'elle se présente sous forme de microsphères, nanosphères, microcapsules ou nanocapsules biodégradables ou encore sous forme de vésicules lipidiques ou de liposomes.

## Claims

1. A vaccination composition designed for the preventive or the curative treatment in humans, by oral antigenic stimulation, of ocular affections, oculo- or urogenital affections of the chlamydiosis type, characterized in that it contains a mixture of freeze-dried elementary bodies and reticulated bodies of Chlamydia trachomatis, including at least one of the serotypes A to K of said species.

2. A composition according to claim 1, designed for the treatment of ocular affections in humans, such as trachoma, characterized in that it contains at least one of the serotypes A to C of the species Chlamydia trachomatis.

3. A composition according to claim 1, designed for the treatment of oculo- or uro-genital affections in humans of the chlamydiosis type, characterized in that it contains at least one of the serotypes D to K of the Chlamydia trachomatis species.

4. A composition according to one of claims 1 to 3, characterized in that it furthermore contains an inert excipient, an immunological adjuvant or stimulant, or furthermore an adjuvant stimulating the activity of macrophages.

5. A composition according to one of claims 1 to 4, characterized in that it is formulated as an oral gastro-resistant dosage form.

6. A composition according to one of claims 1 to 5, characterized in that it is formulated as biodegradable microspheres, nanospheres, microcapsules or nanocapsules or furthermore as lipidic vesicles or liposomes.

## Patentansprüche

1. Impfstoff zur präventiven und kurativen Behandlung von okularen, okulo- oder urogenitalen Affektionen des Chlamydiose-Typs beim Menschen durch orale antigene Stimulation, dadurch gekennzeichnet, dass er ein Gemisch vernetzter und elementarer lyophilisierter Körper von Chlamydia trachomatis mindestens einen der Serotypen A bis K enthält.

2. Impfstoff nach Anspruch 1 zur Behandlung von okularen Affektionen beim Menschen, wie das Trachom, dadurch gekennzeichnet, dass er mindestens einen der Serotypen A bis C der Spezies Chlamydia trachomatis enthält.

3. Impfstoff nach Anspruch 1 zur Behandlung von okulo- oder urogenitalen Affektionen des Chlamydoise-Typs beim Menschen, dadurch gekennzeichnet, dass er mindestens einen der Serotypen D bis K der Spezies Chlamydia trachomatis enthält.

4. Impfstoff nach einem der Ansprüche 1 bis 3, dadurch gekennzeichnet, dass er zusätzlich einen inerten Trägerstoff, ein Adjuvans oder immunologisches Stimulans oder auch ein die Aktivität der Makrophagen stimulierenden Adjuvans enthält.

5. Impfstoff nach einem der Ansprüche 1 bis 4, dadurch gekennzeichnet, dass er in einer oralen gastroresistenten galenischen Form vorliegt.

6. Impfstoff nach einem der Ansprüche 1 bis 5, dadurch gekennzeichnet, dass er in Form von biologisch abbaubaren Mikrokügelchen, Nanokügelchen, Mikrokapseln oder Nanokapseln oder auch in Form von lipidischen Bläschen oder Liposomen vorliegt.
